# EUROPEAN PATENT SPECIFICATION

(11) **EP 4 587 811 B1**
(45) Date of publication and mention of the grant of the patent: **22.04.2026**
(21) Application number: 23786128.1
(22) Date of filing: 13.09.2023
(51) Int. Cl.: G01N 21/15, G01N 21/3504, G01N 21/85, G01N 33/00, G01N 33/497

(54) **DEVICE FOR THE DETECTION OF CARBON DIOXIDE IN A WORKING GAS**
VORRICHTUNG ZUM NACHWEIS VON KOHLENDIOXID IN EINEM ARBEITSGAS
DISPOSITIF DE DETECTION DE DIOXYDE DE CARBONE DANS UN GAZ DE TRAVAIL

(30) Priority: 16.09.2022 IT 202200019005
(43) Date of publication of application: 23.07.2025
(73) Proprietor: Eurosets S.r.l., 41036 Medolla (MO) (IT)
(72) Inventor: CORBELLI, Marco, 41036 Medolla (MO) (IT); CALEFFI, Luca, 41036 Medolla (MO) (IT); FONTANILI, Paolo, 41036 Medolla (MO) (IT); AZZOLINI, Andrea, 41036 Medolla (MO) (IT); GHIRALDI, Andrea, 41036 Medolla (MO) (IT)
(74) Representative: Zoli, Filippo
(86) International application number: PCT/IB2023/059065
(87) International publication number: WO 2024/057215

(56) References cited:
- US-A1- 2002 029 003
- US-A1- 2005 245 836

## Description

### Technical Field

The present invention relates to a device for the detection of carbon dioxide in a working gas, particularly in the medical field.

### Background Art

One of the possible medical applications of this type of device is within extracorporeal blood circulation circuits.

**In** fact, it is well known that during special cardiac surgeries it is necessary to create extracorporeal blood circulation the main purpose of which is to perfuse the vital organs, that is, to supply them with oxygenated blood to ensure their proper function.

Specifically, the systems of known type are provided with an oxygenation device within which the blood drawn from the venous line from the patient is enriched with oxygen before being fed into the patient's arterial line.

Appropriately, the oxygenation device comprises an inlet channel and an outlet channel of a working gas intended to supply oxygen to the blood and/or to remove carbon dioxide therefrom.

Therefore, the amount of carbon dioxide removed by the oxygenator turns out to be a critically important parameter for monitoring the progress of extracorporeal circulation and for assessing the proper oxygenation of the blood.

For this purpose, that is, to detect the amount of carbon dioxide removed from the incoming blood by the patient, measuring devices called, in technical jargon, "capnometers", are generally employed which are connected to the oxygenation device in a fluid-operated manner by means of an appropriate connecting line having the task of conveying the working gas coming out of the oxygenation device into the inlet of the measuring device.

Capnometers can also be used for measuring the amount of carbon dioxide contained in the air exhaled by patients, e.g. during mechanical ventilation treatments or during anesthesia procedures. Such appliances make it possible to determine in real time whether the patient's breathing is occurring properly or not.

In particular, in certain clinical situations wherein carbon dioxide is removed partly by the patient and partly by the oxygenation device positioned along the extracorporeal circulation line, it is of paramount importance to determine the contribution of each of them, that is, to quantify the amount of carbon dioxide removed by the patient and by the oxygenation device.

Capnometers make use of special detection devices comprising a tubular body, of the type of a "cuvette", within which the working gas is conveyed, an emitter of an optical signal adapted to pass through the tubular body and a receiver positioned opposite the emitter and adapted to receive the optical signal.

In this way, through the analysis of the optical signal received from the receiver, e.g. through absorption spectrophotometry of the optical signal, it is possible to trace the amount of carbon dioxide contained in the working gas.

Specifically, the tubular body extends along a relevant longitudinal axis and defines a channel for the flow of the working gas.

Known detection devices also comprise a detection cell comprising two detection elements adapted to allow the passage of the optical signal and which is arranged along the longitudinal axis of the tubular body.

These detection elements are arranged where the side surface of the tubular body is located, so that the optical signal passes through the passage channel of the working fluid in a direction transverse to its longitudinal axis.

In this way, as the gas flows through, the optical signal passes through the gas itself and allows measurement of the amount of carbon dioxide therein.

Known detection devices, however, do have some drawbacks.

In fact, there is also a high concentration of water vapor in the working gas, which vapor tends to condense on the inner walls of the detection device, thus creating accumulation and flow of condensed vapor on the inner walls of the tubular body. When this occurs on the detection elements, the measurement is distorted and corrective factors must therefore be applied in order to obtain a realistic detection.

US 2002/029003 A1 discloses a device for the detection of carbon dioxide in a working gas.

### Description of the Invention

The main aim of the present invention is to devise a device for the detection of carbon dioxide in a working gas which enables proper detection even in the presence of accumulation and flow of condensed vapor on the walls of the tubular body.

Another object of the present invention is to devise a device for the detection of carbon dioxide in a gas which is not altered by the presence of water vapor that may be present on the walls of the tubular body, so as to avoid the application of corrective factors to the detection made.

Another object of the present invention is to devise a device for the detection of carbon dioxide in a working gas which can overcome the aforementioned drawbacks of the prior art within the framework of a simple, rational, easy and effective to use as well as inexpensive solution.

The aforementioned objects are achieved by this device for the detection of carbon dioxide in a working gas having the characteristics of claim 1.

### Brief Description of the Drawings

Other characteristics and advantages of the present invention will become more apparent from the description of a preferred, but not exclusive, embodiment of a device for the detection of carbon dioxide in a working gas, illustrated by way of an indicative, yet non-limiting example in the accompanying tables of drawings in which:
Figure 1 is an axonometric view of a detection device according to the invention, in accordance with a first embodiment;
Figure 2 is a longitudinal sectional view of the device in Figure 1;
Figure 3 is a cross sectional view of the device in Figure 1;
Figure 4 is an axonometric view of a detection device according to the invention, in accordance with a second embodiment;
Figure 5 is a longitudinal sectional view of the device in Figure 4;
Figure 6 is a cross sectional view of the device in Figure 4.

### Embodiments of the Invention

With particular reference to these figures, reference numeral 1 globally denotes a device for the detection of carbon dioxide in a working gas.

In the context of this disclosure, the term "working gas" refers to, e.g., air or oxygen, which escapes from an oxygenation device of extracorporeal blood flow of a type known to the technician in the field. As known, blood oxygenation devices comprise an inlet port of the blood to be oxygenated, an outlet port of the oxygenated blood, an inlet channel and an outlet channel of a working gas, e.g., air or oxygen, intended to supply oxygen to the blood and/or to remove carbon dioxide therefrom. More particularly, the device 1 is connectable to the outlet channel of a blood oxygenation device and/or to a patient's breathing line to monitor the contribution in removing carbon dioxide from the native lung.

The device 1 comprises a tubular body 2 within which a working gas is conveyed. The tubular body 2 extends along a relevant longitudinal axis A and defines a channel 3 for the passage of the working gas. The axial ends of the tubular body 2 are connectable to the outlet channel of a blood oxygenation device or to a patient's breathing line. Depending on whether the tubular body 2 is to be connected to the outlet channel of a blood oxygenation device or to a patient's breathing line, the conformation changes of the axial ends, or of at least of one of them, of the tubular body 2.

The tubular body 2 is made of a polymeric plastic material.

The tubular body 2 comprises two fitting end portions 4 adapted to allow the connection to the outlet channel of a blood oxygenation device.

The device 1 also comprises at least one detection cell 5 associated with the tubular body 2, communicating with the channel 3. The detection cell 5 is positioned at an intermediate position between the two fitting end portions 4.

The detection cell 5 comprises two detection elements 6, opposite each other to define an axis of detection R and adapted to allow the passage of an optical signal, generated by an external measuring device, for the detection of carbon dioxide in the working gas.

The detection cell 5 is adapted to operate in conjunction with the measuring device of the absorption spectrophotometry type, which is configured to emit the optical signal and to acquire the signal resulting from absorption by the working gas. The measuring device comprises, e.g., an emitter and a receiver of the infrared photodiode type and defines a seat adapted to receive the detection cell 5.

The detection elements 6 are made of a different material from the detection cell **5. In** detail, the detection elements 6 are made of a material which is transparent to infrared radiation. For example, the detection elements 6 are made of sapphire glass.

The detection cell 5, on the other hand, is made of a polymeric plastic material. The polymeric plastic material can be of the same type as the tubular body 2 or of a different type, such as the type of an elastomer.

Thus, the device 1 allows the detection of carbon dioxide during the passage of the working gas through the tubular body 2.

According to the invention, the detection cell 5 protrudes from the tubular body 2 in a direction transverse to the longitudinal axis A. Therefore, the detection cell 5 extends along a direction transverse to the longitudinal axis A.

**In** more detail, the detection cell 5 protrudes from the tubular body 2 in a direction perpendicular to the longitudinal axis A. The detection cell 5 extends orthogonally with respect to the longitudinal axis A and juts outwards from the tubular body 2.

Thus, the detection cell 5 defines a chamber 7 communicating with the channel 3 in a fluid-operated manner and offset therefrom, that is arranged so that it is not intercepted by its longitudinal axis A. **In** actual facts, the chamber 7 is misaligned with respect to the channel 3.

The detection cell 5 then causes a split of the working gas flow into a main flow transiting in the channel 3 and a secondary flow transiting in the chamber 7.

Where the detection cell 5 is located, the secondary flow exits the channel 3 and enters the chamber 7, where it intercepts the optical signal, enables measurement and later re-enters the channel 3.

The misaligned position of the chamber 7 means that the detection cell 5 is not occupied by condensate. **In** fact, the condensate consists of water vapor droplets which, being heavier than carbon dioxide and other exhaled gases, is not deflected by the secondary flow but is deposited on the walls of the tubular body 2.

The detection cell 5 is not intercepted by the condensate passing through the channel 3, so the optical signal passing through the detection cell itself is not affected by the condensate interference, thus allowing for optimal carbon dioxide detection.

Advantageously, the axis of detection R passes externally to the channel 3. The axis of detection R, therefore, does not pass through the channel 3 but only through the chamber 7. The detection is, therefore, carried out on the gas present in the chamber 7, that is, on the secondary flow and not on the main flow.

Specifically, the axis of detection R is substantially orthogonal to the longitudinal axis A. Detection is substantially carried out orthogonally to the direction of flow of the working gas.

The detection elements 6 are opposite each other with respect to a plane of symmetry P passing through the longitudinal axis A.

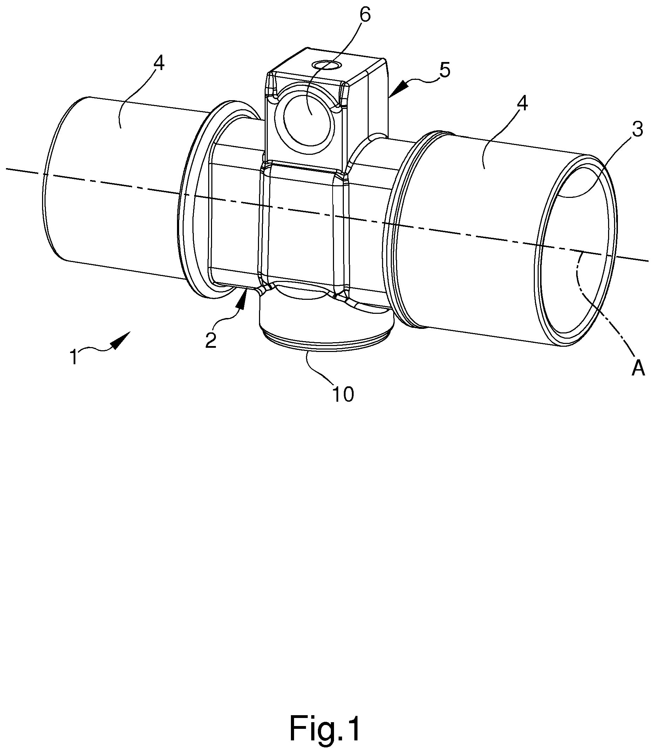

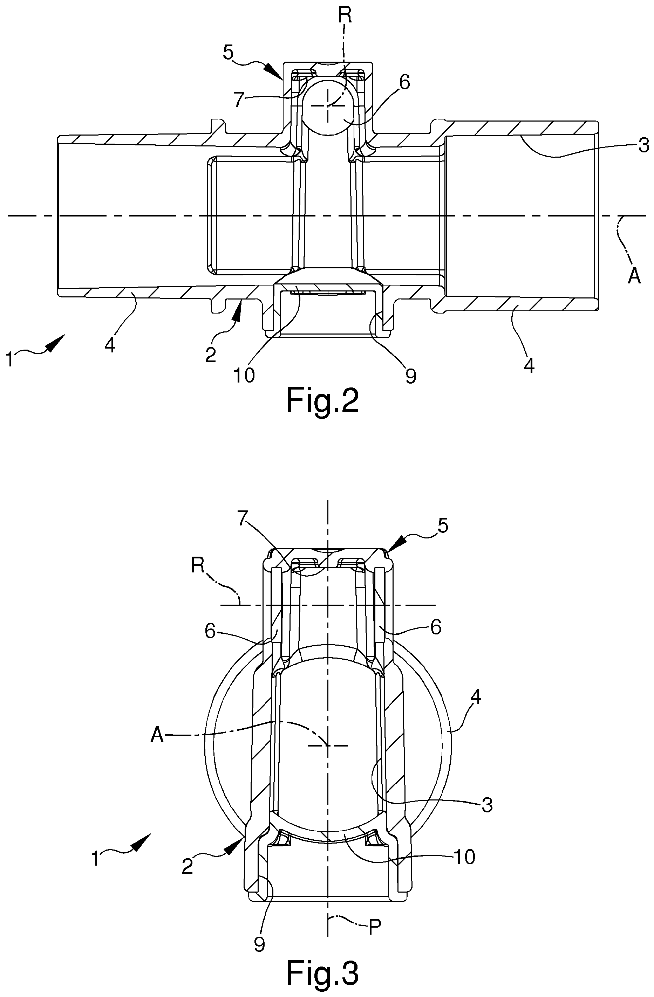

**In** accordance with a first embodiment shown in Figures 1 to 3, the detection cell 5 and the tubular body 2 are made in a single body piece. **In** this first embodiment, as described in more detail below, the detection elements 6 are preferably co-molded with the single body piece consisting of the tubular body 2 and the detection cell 5.

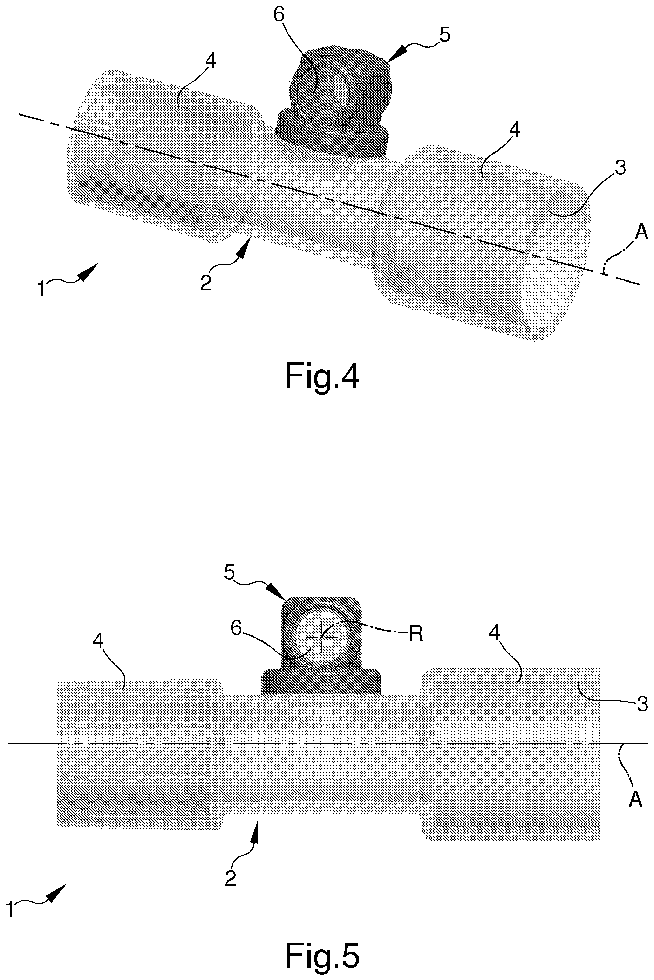

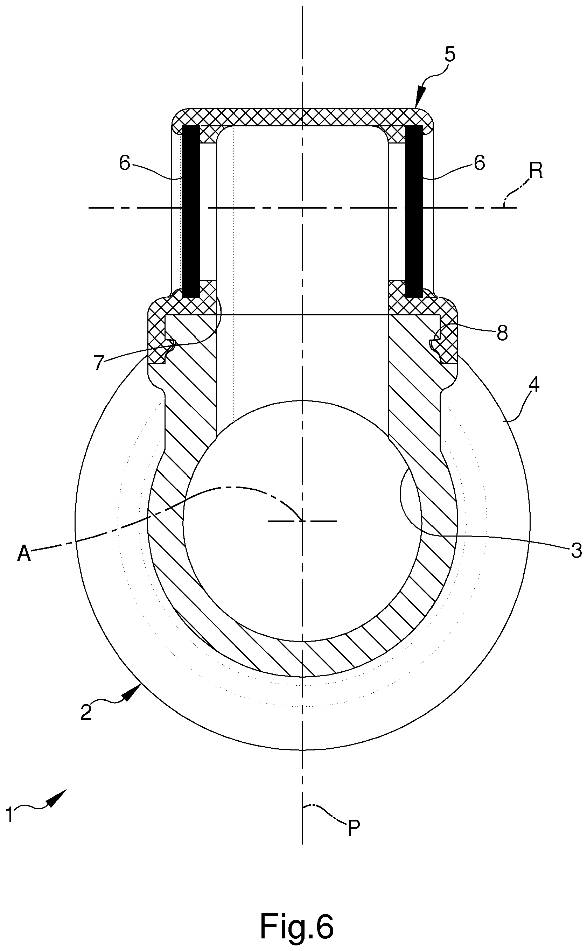

**In** accordance with a second embodiment shown in Figures 4 through 6, however, the detection cell 5 and the tubular body 2 are made in separate body pieces. For this purpose, the device 1 comprises fastening means 8 located between the detection cell 5 and the tubular body 2.

Advantageously, the fastening means 8 are of the interlocking type.

**In** this way, the detection cell 5 and the tubular body 2 can be assembled in a very cheap manner.

It cannot, however, be ruled out that the fastening means may be of a different type known to the technician in the field.

The device 1 in accordance with the second embodiment also comprises sealing means adapted to ensure a tight seal between the detection cell 5 and the tubular body 2 as well as between the detection elements 6 and the detection cell 5. Appropriately, if the detection cell 5 is made of an elastically deformable material, the sealing means coincide with the fastening means 8.

The sealing means comprise, e.g., a seal made of elastically deformable material.

According to a further aspect, the present invention also relates to a process according to claim 10 for the manufacture of a device 1 for the detection of carbon dioxide in a working gas in accordance with one or more of the embodiments described above.

The process according to the invention comprises the following stages:
- supplying a mould having a profile substantially complementary to the tubular body 2;
- supplying a forming jig having a profile at least partly complementary to the detection cell 5;
- plastic molding of the tubular body 2 by means of the mould; and
- forming the detection cell 5 by means of the forming jig.

In accordance with the first embodiment, the forming stage is carried out at the same time as the molding stage so that the detection cell 5 can be defined as a single body piece with the tubular body 2.

In detail, the forming stage of the detection cell 5 is carried out by inserting the forming jig through the tubular body 2 during the relevant molding. The forming jig is, in actual facts, the "male" tool which is inserted inside the mould relating to the tubular body 2 to achieve the forming of the detection cell 5.

The forming stage causes the definition of a passage opening 9 in the tubular body 2, through which the forming jig is inserted and removed.

For this purpose, the process comprises a closing stage of the passage opening 9. In detail, the closing stage is carried out by applying a plug element 10.

It cannot, however, be ruled out that the closing stage may be carried out in a different manner, such as by plastic welding.

Advantageously, the forming jig is adapted to support the detection elements 6 during the forming stage. In actual facts, the detection elements 6 are applied to the detection cell 5 and incorporated by it at the same time as its forming during the molding stage.

In accordance with the second embodiment, on the other hand, the forming stage of the detection cell 5 is carried out separately to the molding stage. In such a case, the detection cell 5 can be made of a different material from the tubular body 2, preferably of an elastically deformable material.

Appropriately, the forming of the detection cell 5 also involves the definition of the housing seats of the detection elements 6 which are inserted into them after forming is completed, e.g. by taking advantage of the elastic deformability of the material from which the detection cell 5 is made.

Conveniently, the forming stage of the detection cell 5 comprises a step of definition of the fastening means 8 on the detection cell itself. The forming jig substantially comprises a portion shaped in a complementary manner to the corresponding fastening means.

The process also comprises an attaching stage of the detection cell 5 to the tubular body 2.

The attaching stage then comprises at least one step of coupling the detection cell 5 to the tubular body 2 by interlocking.

Depending on the conformation of the fastening means 8, the attaching stage may also comprise a step of definition of the fastening means 8 on the tubular body 2 which are complementary to the fastening means defined on the detection cell 5. The step of definition can be carried out at the same time as the molding stage or separately thereto, such as e.g. by machining.

It has in practice been ascertained that the described invention achieves the intended objects and in particular the fact is emphasized that the present device for the detection of carbon dioxide in a working gas enables correct detection even in the presence of condensed vapor on the walls of the tubular body.

The presence of a detection cell protruding from the tubular body makes it possible to carry out a detection of the carbon dioxide contained in the working gas that is not affected by the possible presence of condensate within the tubular body itself, so as to obtain as realistic a reading as possible and that does not require the use of correction factors.

The device for the detection of carbon dioxide according to the invention can be applied either to a patient's breathing line or to the outlet channel of a blood oxygenation device.

## Claims

1. Device (1) for the detection of carbon dioxide in a working gas comprising:
- a tubular body (2) within which a working gas is conveyed, which extends along a relevant longitudinal axis (A) and defines a channel (3) for the passage of said working gas;
- at least one detection cell (5) associated with said tubular body (2), communicating with said channel (3) and comprising two detection elements (6), opposite each other to define an axis of detection (R) and adapted to allow the passage of an optical signal, generated by an external measuring device, for the detection of carbon dioxide in said working gas, said detection cell (5) defining a chamber (7) communicating with said channel (3) in a fluid-operated manner for the passage of part of said working gas;
**characterized by** the fact that said detection cell (5) is protruding from said tubular body (2) in a direction transverse to said longitudinal axis (A), said chamber (7) being misaligned with respect to said channel (3).

2. Device (1) according to claim 1, **characterized by** the fact that said detection cell (5) is protruding from said tubular body (2) in a direction perpendicular to said longitudinal axis (A).

3. Device (1) according to one or more of the preceding claims, **characterized by** the fact that said axis of detection (R) passes externally to said channel (3).

4. Device (1) according to one or more of the preceding claims, **characterized by** the fact that said axis of detection (R) is substantially orthogonal to said longitudinal axis (A).

5. Device (1) according to one or more of the preceding claims, **characterized by** the fact that said detection elements (6) are opposite each other with respect to a plane of symmetry (P) passing through said longitudinal axis (A).

6. Device (1) according to one or more of the preceding claims, **characterized by** the fact that said detection cell (5) and said tubular body (2) are made in a single body piece.

7. Device (1) according to one or more of the preceding claims, **characterized by** the fact that said detection cell (5) and said tubular body (2) are made in separate bodies and by the fact that said device (1) comprises fastening means (8) located between said detection cell (5) and said tubular body (2).

8. Device (1) according claim 7, **characterized by** the fact that said fastening means (8) are of the interlocking type.

9. Device (1) according to claim 7 and/or 8, **characterized by** the fact that it comprises sealing means associated with said fastening means (8).

10. Process for the manufacture of a device (1) for the detection of carbon dioxide in a working gas according to one or more of the preceding claims, **characterized by** the fact that it comprises the following stages:
- supplying a mould having a profile substantially complementary to said tubular body (2);
- supplying a forming jig having a profile at least partly complementary to said detection cell (5);
- plastic molding of said tubular body (2) by means of said mould; and
- forming said detection cell (5) by means of said forming jig.

11. Process according to claim 10, **characterized by** the fact that said forming stage is carried out at the same time as said molding stage so that said detection cell (5) is defined as a single body piece with said tubular body (2).

12. Process according to claim 10, **characterized by** the fact that said forming jig is adapted to support said detection elements (6) during said forming stage, said detection elements (6) being made of a different material from said detection cell (5).

13. Process according to claim 10, **characterized by** the fact that said forming stage is carried out separately to said molding stage, said detection cell (5) being made of a different material from said tubular body (2).

14. Process according to claim 13, **characterized by** the fact that said forming stage comprises a step of definition of said fastening means (8) on said detection cell (5).

15. Process according to claim 14, **characterized by** the fact that it comprises a stage of attaching said detection cell (5) to said tubular body (2) comprises at least one step of coupling said detection cell (5) to said tubular body (2) by interlocking.

## Patentansprüche

1. Vorrichtung (1) zum Detektieren von Kohlenstoffdioxid in einem Arbeitsgas, umfassend:
- einen rohrförmigen Körper (2), in dem ein Arbeitsgas befördert wird, der sich entlang einer relevanten Längsachse (A) erstreckt und einen Kanal (3) für den Durchgang des Arbeitsgases definiert;
- mindestens eine Detektionszelle (5), die dem rohrförmigen Körper (2) zugeordnet ist, mit dem Kanal (3) in Verbindung steht und zwei Detektionselemente (6) umfasst, die einander gegenüberliegen, um eine Detektionsachse (R) zu definieren, und dazu ausgebildet sind, den Durchgang eines optischen Signals zu ermöglichen, das von einer externen Messvorrichtung erzeugt wird, um Kohlenstoffdioxid in dem Arbeitsgas zu detektieren, wobei die Detektionszelle (5) eine Kammer (7) definiert, die mit dem Kanal (3) in einer fluidbetätigten Weise für den Durchgang eines Teils des Arbeitsgases in Verbindung steht;
**dadurch gekennzeichnet, dass** die Detektionszelle (5) aus dem rohrförmigen Körper (2) in einer Richtung quer zu der Längsachse (A) herausragt, wobei die Kammer (7) in Bezug auf den Kanal (3) dejustiert ist.

2. Vorrichtung (1) nach Anspruch 1, **dadurch gekennzeichnet, dass** die Detektionszelle (5) aus dem rohrförmigen Körper (2) in einer Richtung senkrecht zu der Längsachse (A) herausragt.

3. Vorrichtung (1) nach einem oder mehreren der vorhergehenden Ansprüche, **dadurch gekennzeichnet, dass** die Detektionsachse (R) außerhalb des Kanals (3) verläuft.

4. Vorrichtung (1) nach einem oder mehreren der vorhergehenden Ansprüche, **dadurch gekennzeichnet, dass** die Detektionsachse (R) im Wesentlichen orthogonal zur Längsachse (A) verläuft.

5. Vorrichtung (1) nach einem oder mehreren der vorhergehenden Ansprüche, **dadurch gekennzeichnet, dass** die Detektionselemente (6) einander gegenüberliegen in Bezug auf eine Symmetrieebene (P), die durch die Längsachse (A) verläuft.

6. Vorrichtung (1) nach einem oder mehreren der vorangehenden Ansprüche, **dadurch gekennzeichnet, dass** die Detektionszelle (5) und der rohrförmige Körper (2) aus einem einzigen Körperteil bestehen.

7. Vorrichtung (1) nach einem oder mehreren der vorhergehenden Ansprüche, **dadurch gekennzeichnet, dass** die Detektionszelle (5) und der rohrförmige Körper (2) in getrennten Körpern ausgeführt sind und dass die Vorrichtung (1) Befestigungsmittel (8) umfasst, die zwischen der Detektionszelle (5) und dem rohrförmigen Körper (2) angeordnet sind.

8. Vorrichtung (1) nach Anspruch 7, **dadurch gekennzeichnet, dass** die Befestigungsmittel (8) vom verriegelnden Typ sind.

9. Vorrichtung (1) nach Anspruch 7 und/oder 8, **dadurch gekennzeichnet, dass** sie Dichtungsmittel umfasst, die den Befestigungsmitteln (8) zugeordnet sind.

10. Verfahren zur Herstellung einer Vorrichtung (1) zum Detektieren von Kohlenstoffdioxid in einem Arbeitsgas nach einem oder mehreren der vorhergehenden Ansprüche, **dadurch gekennzeichnet, dass** es die folgenden Schritte umfasst:
- Bereitstellen einer Form mit einem Profil, das im Wesentlichen komplementär zu dem rohrförmigen Körper (2) ist;
- Bereitstellen einer Formvorrichtung mit einem Profil, das zumindest teilweise komplementär zu der Detektionszelle (5) ist;
- plastisches Abformen des rohrförmigen Körpers (2) mit Hilfe der Form; und
- Formen der Detektionszelle (5) mit Hilfe der Formvorrichtung.

11. Verfahren nach Anspruch 10, **dadurch gekennzeichnet, dass** der Schritt des Formens gleichzeitig mit dem Schritt des Abformens durchgeführt wird, so dass die Detektionszelle (5) mit dem rohrförmigen Körper (2) als ein einziges Körperteil definiert ist.

12. Verfahren nach Anspruch 10, **dadurch gekennzeichnet, dass** die Formvorrichtung dazu ausgebildet ist, die Detektionselemente (6) während des Schritts des Formens zu tragen, wobei die Detektionselemente (6) aus einem anderen Material als die Detektionszelle (5) hergestellt sind.

13. Verfahren nach Anspruch 10, **dadurch gekennzeichnet, dass** der Schritt des Formens getrennt von dem Schritt des Abformens durchgeführt wird, wobei die Detektionszelle (5) aus einem anderen Material als der rohrförmige Körper (2) hergestellt ist.

14. Verfahren nach Anspruch 13, **dadurch gekennzeichnet, dass** der Schritt des Formens einen Schritt des Definierens der Befestigungsmittel (8) an der Detektionszelle (5) umfasst.

15. Verfahren nach Anspruch 14, **dadurch gekennzeichnet, dass** es einen Schritt des Anbringens der Detektionszelle (5) an dem rohrförmigen Körper (2) umfasst, der mindestens einen Schritt des Koppelns der Detektionszelle (5) mit dem rohrförmigen Körper (2) durch Verriegelung umfasst.

## Revendications

1. - Dispositif (1) pour la détection de dioxyde de carbone dans un gaz de travail, comprenant :
- un corps tubulaire (2) à l'intérieur duquel est transporté un gaz de travail, qui s'étend le long d'un axe longitudinal correspondant (A) et définit un canal (3) pour le passage dudit gaz de travail ;
- au moins une cellule de détection (5) associée audit corps tubulaire (2), communiquant avec ledit canal (3) et comprenant deux éléments de détection (6), opposés l'un à l'autre pour définir un axe de détection (R) et aptes à permettre le passage d'un signal optique, généré par un dispositif de mesure externe, pour la détection de dioxyde de carbone dans ledit gaz de travail, ladite cellule de détection (5) définissant une chambre (7) communiquant avec ledit canal (3) d'une manière actionnée par fluide pour le passage d'une partie dudit gaz de travail ;
**caractérisé par le fait que** ladite cellule de détection (5) fait saillie à partir dudit corps tubulaire (2) dans une direction transversale audit axe longitudinal (A), ladite chambre (7) étant désalignée par rapport audit canal (3).

2. - Dispositif (1) selon la revendication 1, **caractérisé par le fait que** ladite cellule de détection (5) fait saillie à partir dudit corps tubulaire (2) dans une direction perpendiculaire audit axe longitudinal (A).

3. - Dispositif (1) selon l'une ou plusieurs des revendications précédentes, **caractérisé par le fait que** ledit axe de détection (R) passe à l'extérieur dudit canal (3) .

4. - Dispositif (1) selon l'une ou plusieurs des revendications précédentes, **caractérisé par le fait que** ledit axe de détection (R) est sensiblement orthogonal audit axe longitudinal (A).

5. - Dispositif (1) selon l'une ou plusieurs des revendications précédentes, **caractérisé par le fait que** lesdits éléments de détection (6) sont opposés l'un à l'autre par rapport à un plan de symétrie (P) passant par ledit axe longitudinal (A).

6. - Dispositif (1) selon l'une ou plusieurs des revendications précédentes, **caractérisé par le fait que** ladite cellule de détection (5) et ledit corps tubulaire (2) sont réalisés en une pièce monobloc.

7. - Dispositif (1) selon l'une ou plusieurs des revendications précédentes, **caractérisé par le fait que** ladite cellule de détection (5) et ledit corps tubulaire (2) sont réalisés en corps séparés et **par le fait que** ledit dispositif (1) comprend des moyens de fixation (8) situés entre ladite cellule de détection (5) et ledit corps tubulaire (2).

8. - Dispositif (1) selon la revendication 7, **caractérisé par le fait que** lesdits moyens de fixation (8) sont du type à verrouillage réciproque.

9. - Dispositif (1) selon la revendication 7 et/ou 8, **caractérisé par le fait qu'**il comprend des moyens d'étanchéité associés auxdits moyens de fixation (8).

10. - Procédé de fabrication d'un dispositif (1) pour la détection de dioxyde de carbone dans un gaz de travail selon l'une ou plusieurs des revendications précédentes, **caractérisé par le fait qu'**il comprend les étapes suivantes :
- fournir un moule ayant un profil sensiblement complémentaire dudit corps tubulaire (2) ;
- fournir un gabarit de formage ayant un profil au moins partiellement complémentaire de ladite cellule de détection (5) ;
- effectuer un moulage plastique dudit corps tubulaire (2) au moyen dudit moule ; et
- former ladite cellule de détection (5) au moyen dudit gabarit de formage.

11. - Procédé selon la revendication 10, **caractérisé par le fait que** ladite étape de formage est réalisée en même temps que ladite étape de moulage de telle sorte que ladite cellule de détection (5) est définie comme une pièce monobloc avec ledit corps tubulaire (2).

12. - Procédé selon la revendication 10, **caractérisé par le fait que** ledit gabarit de formage est apte à porter lesdits éléments de détection (6) pendant ladite étape de formage, lesdits éléments de détection (6) étant réalisés dans un matériau différent par rapport à ladite cellule de détection (5).

13. - Procédé selon la revendication 10, **caractérisé par le fait que** ladite étape de formage est réalisée séparément de ladite étape de moulage, ladite cellule de détection (5) étant réalisée dans un matériau différent par rapport audit corps tubulaire (2).

14. - Procédé selon la revendication 13, **caractérisé par le fait que** ladite étape de formage comprend une étape de définition desdits moyens de fixation (8) sur ladite cellule de détection (5).

15. - Procédé selon la revendication 14, **caractérisé par le fait qu'**il comprend une étape de fixation de ladite cellule de détection (5) audit corps tubulaire (2) qui comprend au moins une étape de couplage de ladite cellule de détection (5) audit corps tubulaire (2) par verrouillage réciproque.
